(19) Europäisches Patentamt
European Patent Office
Office européen des brevets

(11) EP 2 275 424 A1

(12) EUROPEAN PATENT APPLICATION

(43) Date of publication:
**19.01.2011 Bulletin 2011/03**

(51) Int Cl.:
***C07D 477/20*** *(2006.01)* ***A61K 31/407*** *(2006.01)*
***A61P 31/04*** *(2006.01)*

(21) Application number: **10169837.1**

(22) Date of filing: **16.07.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **17.07.2009 EP 09165773**

(71) Applicant: **Sandoz AG**
**4056 Basel (CH)**

(72) Inventors:
- **Hotter, Andreas**
  **6250 Kundl (AT)**
- **Bartsch, Susanne**
  **6250 Kundl (AT)**
- **Backes, Alexander**
  **6250 Kundl (AT)**

(74) Representative: **Wichmann, Hendrik et al**
**Wuesthoff & Wuesthoff**
**Schweigerstrasse 2**
**81541 München (DE)**

# (54) Doripenem crystallization process

(57) The present invention provides an improved crystallization process for Doripenem Monohydrate in high purity and with defined physical properties. The crystallization process comprises the steps of dissolving Doripenem in water by adjusting a basic pH followed by adjusting an acidic pH to induce crystallization of Doripenem Dihydrate. The present invention is especially suitable for the manufacture of sterile Doripenem Monohydrate (form IV).

EP 2 275 424 A1

## Description

### FIELD OF THE INVENTION

The present invention provides an improved crystallization process for Doripenem Monohydrate in high purity and with defined physical properties. The crystallization process comprises the steps of dissolving Doripenem in water by adjusting a basic pH followed by adjusting an acidic pH to induce crystallization of Doripenem Dihydrate. The present invention is especially suitable for the manufacture of sterile Doripenem Monohydrate (form IV).

### BACKGROUND OF THE INVENTION

Doripenem, (4R,5S,6S)-3-[[(3S,5S)-5-[[(Aminosulfonyl)amino]methyl]-3-pyrrolidinyl]thio]-6-[(1*R*)-1-hydroxyethyl]-4-methyl-7-oxo-1-azabicyclo[3.2.0]hept-2-ene-2-carboxylic acid is a new parenteral carbapenem antibiotic possessing a sulfamoylaminoethyl-pyrrolidinylthin group as side chain at position 2 which enhances its activity against non-fermentative Gram-negative bacilli compared to other carbapenems. Equal to Meropenem and Ertapenem, Doripenem is stable to the enzyme Dehydropeptidase-1 (DHP-1) due to the presence of a 1-β-methyl moiety on the carbapenem nucleus. The trans-α-1-hydroxyethyl substituent at position 6 is responsible for the β-lactamase stability. The chemical structure of Doripenem is shown as Formula A.

**Formula A: Chemical structure of Doripenem**

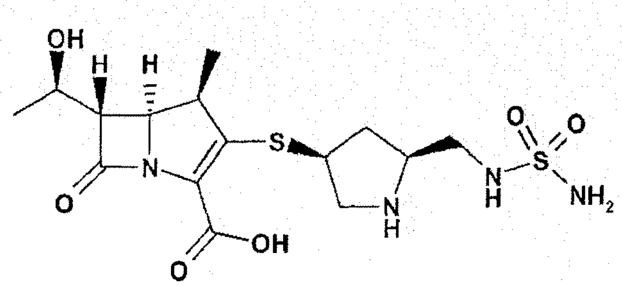

All β-lactam antimicrobial agents including the carbapenems exhibit bactericidal activity by binding to penicillin-binding proteins (PBPs). The binding of the β-lactam molecule to the PBPs prevents bacteria from completing transpeptidation (cross-linking) of peptidoglycan strands, thus preventing the synthesis of an intact bacterial cell wall. Doripenem shows *in vitro* activity against Gram-positive aerobic bacteria e.g. methicillin-susceptible Staphylococcus aureus, Gram-negative aerobic bacteria e.g. Escherichia coli and against anaerobic bacteria such as Bacteroides fragilis.

Doripenem is available on the market as powder for solution for infusion, the brand name is Doribax® and the originator is Shionogi & Co. The finished dosage form contains Doripenem Monohydrate and does not contain any excipients. Currently Doripenem is indicated for nosocomial pneumonia, complicated intra-abdominal infections and complicated urinary tract infections. It is excreted almost exclusively in urine and predominantly as unchanged drug. [Merck Index 14th Edition, monograph number 10227, CAS Registry Number 148016-81-3; Zhanel GG et al., Comparative review of the carbapenems. Drugs 2007; 67 (7): 1027-1052; Brown SD, Traczewski MM., Comparative in vitro antimicrobial activity of a new carbapenem, Doripenem: tentative disc diffusion criteria and quality control. J. Antimicrob. Chemother. 2005 Jun; 55 (6): 944-949; Pillar CM et al. In vitro activity of Doripenem, a carbapenem for the treatment of challenging infections caused by gram-negative bacteria, against recent clinical isolates from the United States. Antimicrob. Agents Chemother., 2008 Dec; 52 (12): 4388-4399; Jones RN et al. Doripenem (S-4661), a novel carbapenem: comparative activity against contemporary pathogens including bactericidal action and preliminary in vitro methods evaluations. J. Antimicrob. Chemother. 2004 Jul; 54 (1): 144-154; Cirillo I et al. Disposition, metabolism, and excretion of [14C]Doripenem after a single 500-milligramm intravenous infusion in healthy men. Antimicrob. Agents Chemother., 2008 Oct, 52 (10): 3478-3483;]

Doripenem is described in EP 557122. However, EP 557122 describes only examples in which amorphous Doripenem was isolated. EP 758651 describes crystalline forms I and II of Doripenem as well as methods of preparing the same. EP 1270575 discloses crystalline forms III and IV of Doripenem and processes of preparing them. EP 1886682 relates to a method of preparing an aqueous Doripenem solution by continuous heating. WO 2006/117763 discloses processes for the preparation of Doripenem and for the preparation of Doripenem in amorphous form.

The present invention provides an improved crystallization process for Doripenem Monohydrate in high purity and with defined physical properties.

## SUMMARY OF THE INVENTION

The present invention provides an improved crystallization process for Doripenem Monohydrate in high purity and with defined physical properties comprising the steps of:

(a) suspending Doripenem in water,
(b) dissolving Doripenem at about pH 10 by the addition of a base,
(c) optionally filtering the solution through a micron filter,
(d) crystallizing Doripenem at about pH 5 by the addition of an acid,
(e) optionally adding an organic solvent and
(f) isolating and drying the obtained crystals to obtain Doripenem form IV (Monohydrate).

Other objects, features, advantages and aspects of the present invention will become apparent to those of skill from the following description. It should be understood, however, that the description and the following specific examples, while indicating preferred embodiments of the invention, are given by way of illustration only. Various changes and modifications within the spirit and scope of the disclosed invention will become readily apparent to those skilled in the art from reading the description and from reading the other parts of the present disclosure.

## BRIEF DESCRIPTION OF THE DRAWING

Figure 1: Doripenem decomposition curve at pH 10

## DETAILED DESCRIPTION OF THE INVENTION

The present invention provides an improved crystallization process for Doripenem in high purity and with defined physical properties.
A crystallization process comprising the steps of:

(a) suspending Doripenem in water,
(b) dissolving Doripenem at about pH 8 -12 by the addition of a base,
(c) optionally filtering the solution through a micron filter,
(d) crystallizing Doripenem at about pH 3 - 6 by the addition of an acid,
(e) optionally adding an organic solvent and
(f) isolating and drying the obtained crystals to obtain the Monohydrate of Doripenem

is subject matter of the present invention.
In step (a) the starting material is suspended in water, optionally in admixture with another solvent. Suitable other solvents are ketones e.g. acetone or ethylmethylketone or alcohols such as methanol, ethanol, propanol and isopropanol. The starting material can be any kind of crystalline forms of Doripenem or mixtures thereof, amorphous Doripenem or mixtures of amorphous and crystalline Doripenem. As starting material for the novel crystallization process of the present invention crude Doripenem as obtained from the synthesis procedures may be used.
The starting material is preferably used in a concentration in the range from about 10 to 200 g/L, more preferably from about 30 to 100 g/L, most preferably in a concentration from about 50 to 60 g/L.
The temperature of the suspension in step (a) may range from about 0 °C to 30 °C, preferably from about 0 °C to 25 °C.
In step (b) a pH of about 8 - 12, preferably a pH of about 9 - 11, most preferably a pH of about 10 is adjusted in order to obtain a clear solution.
Any suitable base can be used in step (b) in order to adjust the pH. E.g. an ammonium source, e.g. an aqueous $NH_3$ solution, ammonium carbonate or ammonium hydroxide; an organic amine, e.g. triethylamine, diisopropylamine, tert.-butylamine, diethylamine as examples of primary, secondary or tert.-amines; cyclic amines, e.g. pyrrolidine or piperidine; an alkali or earth alkali carbonate or bicarbonate, e.g. lithium, potassium or sodium bicarbonate, or lithium, sodium or potassium carbonate, an alkali or earth alkali hydroxide, e.g. sodium, potassium or lithium hydroxide. In the most preferred embodiment sodium hydroxide is used. The concentration of the sodium hydroxide solution preferably is in the range from about 5 - 50 %. Most preferably the concentration is 20 %.
The temperature of the suspension in step (b) is the same as in step (a) and may range from 0 °C to 30 °C,

preferably from 0 °C to 25 °C.

In step (d) a pH of about 3 - 6, preferably a pH of about 4 - 6, more preferably a pH of about 4.5 - 5.5, most preferably a pH of about 5 is adjusted in order to initiate crystallization of Doripenem.

Any suitable acid can be used in step (d) in order to adjust the pH. Typical acids include inorganic acids, e.g. hydrochloric acid, sulfuric acid, phosphoric acid and the like. Suitable organic acids include sulfonic acids, e.g. methanesulfonic acid or benzenesulfonic acid, organic mono acids, e.g. acetic acid or organic bis acids, e.g. oxalic acid or fumaric acid. Most preferably hydrochloric acid is used. The concentration of hydrochloric acid preferably is in the range from about 1 - 37 %. Most preferably the concentration is 10 %.

The crystallization temperature in step (d) is the same as in steps (a) and (b) and may range from about 0 °C to 30 °C, preferably from about 0 °C to 25 °C.

Stirring is conducted during crystallization, as long as the stirring is carried out in a manner which is gentle enough, so that it does not interfere with crystallization.

When crystallization took place the suspension may be stirred at the adjusted temperature until the main part of the material crystallized. The exact stirring time can vary. The stirring time may be in the range from about 0.5 to 24 hours, preferably from about 1 to 3 hours.

To improve the yield the suspension may be further stirred at about 0 to 5 °C. The stirring time at about 0 to 5 °C can vary. The stirring time may be in the range from about 0.5 to 24 hours, preferably from about 1 to 3 hours.

To complete crystallization an antisolvent may be added to the suspension. Suitable antisolvents are ketones e.g. acetone or ethylmethylketone or alcohols such as methanol, ethanol, propanol and isopropanol. Most preferably isopropanol is used. The isopropanol concentration may be in the range from about 20 - 100 % (v/v), preferably from about 30 - 70 % (v/v), most preferably the concentration is about 40 % (v/v) compared to the amount of water used in the initial dissolution/suspension step of Doripenem. Part of the solvent may be already present in the dissolution step.

In step (f) Doripenem can be isolated from the mixture by any conventional methods such as filtration, centrifugation or evaporation of the solvent.

According to the present crystallization process Doripenem Dihydrate crystallizes. By drying the obtained material at a temperature of about 50 °C under vacuum the desired monohydrate in pure form is obtained. This procedure is described in EP 1270575.

The poor stability of β-lactam antibiotics especially carbapenem antibiotics with regard to hydrolysis of the β-lactam ring is a known problem.

It is known e.g. from EP 1886682 that aqueous Doripenem solutions are unstable to heat. A prominent degradation product is e.g. ring-opened Doripenem (RO-Doripenem) with a chemical structure as given in Formula B.

**Formula B: Chemical structure of RO-Doripenem**

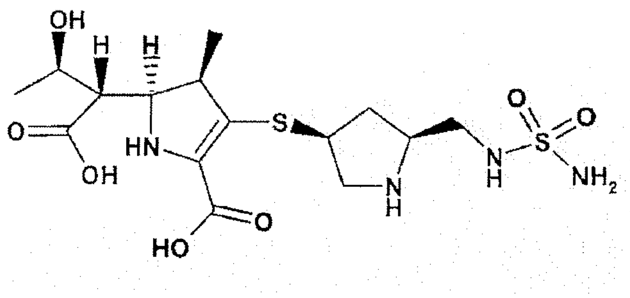

Figures 2 - 4 of EP 1886682 show Doripenem decomposition curves at 40 - 60 °C. The Doripenem remaining rate after 120 minutes at 40 °C is < 0.95, the Doripenem remaining rate at 50 °C after 120 minutes is < 0.90 and the Doripenem remaining rate at 60 °C after 120 minutes is < 0.70 according to EP 1886682.

Commercially available Doripenem Monohydrate contains about 0.2 % (weight-%) of the hydrolyzed Doripenem derivative of Formula B (RO-Doripenem). The control of this degradation product in the manufacture of the API is therefore crucial to keep levels of this degradation product low in the finished dosage form, as this degradation product is inactive.

The chemical stability of Doripenem:

Surprisingly the inventors of the present invention found that Doripenem remains stable in basic aqueous solution even at relatively high pH. Decomposition of Doripenem in aqueous basic solution was determined and the Doripenem decomposition curve is displayed in Figure 1. After 120 minutes in aqueous solution at pH 10.0 the Doripenem

remaining rate did not change and even after 540 minutes at pH 10.0 the Doripenem remaining rate was still the same.

In Example 1 of EP 1270575 a Doripenem solution is prepared by heating a suspension of Doripenem in ion-exchanged water to about 50 - 55 °C. The crystallization process of the present invention was compared with the process of Example 1 of EP 1270575 concerning RO-Doripenem. Table 1 displays that already in a small scale, where the aqueous Doripenem solution is exposed to heat only for a short period, the process of EP 1270575 leads to material with a higher RO-Doripenem content. In contrast the present process leads to highly pure material with only 0.1 % (weight-%) of RO-Doripenem (determined by HPLC at 210 nm against a solution of reference standard of Doripenem representing a content of 1 weight-%, analytical method and calculation see below). The experimental conditions for this comparison are disclosed in Example 7 and Reference Example 1.

**Table 1:** Comparison of impurities following reference

| | **Experiment 7 according to the present process** | **Reference example 1 according to Example 1 of** EP 1270575 |
|---|---|---|
| **Dissolved at** | **pH 10** | **50 - 55 °C** |
| **Doripenem as is [weight-%]** | 95.5 % | 94.3 % |
| **RO-Doripenem [weight-%]** | 0.1% | 0.2 % |

In EP 1886682 the problem of decomposition of an aqueous Doripenem solution by heat is solved by continuous heating of a Doripenem suspension. This process asks for a dissolving apparatus consisting of a container for accommodating a suspension, a continuous heating and dissolving equipment and a container for recovering an aqueous solution as described in said patent application. By this process small amounts of the Doripenem suspension are dissolved successively. However, this process has the disadvantage of being time- and space-consuming. Furthermore this process asks for special expensive equipment.

The present invention comprises a fast, easy to perform, time- space- and money-saving crystallization process with no need for special equipment. In the present process a Doripenem solution is obtained by adjusting a pH of about 10 by the addition of a base. The temperature may be in the range from about 0 °C to 30 °C, which means that according to the present crystallization process Doripenem is not exposed to heat. Therefore undesired degradation products that are formed during exposure of an aqueous Doripenem solution to heat are avoided by the present process. Crystallization is initiated by adjusting the pH to the isoelectric point of Doripenem being around pH 5.

The present invention thus provides a process for the manufacture of Doripenem Monohydrate with levels of compound of formula B of about 0.1 % (weight-%) or less. If desirable, the dissolution/crystallization process may be performed in a continuous manner.

In another embodiment there is provided an advantageous crystallization process for Doripenem Monohydrate with respect to the control of desired physical properties.

The present invention provides a crystallization process for Doripenem with the freedom to select the temperature of the crystallization. The freedom to select the temperature for the crystallization step is due to the option of acidifying the alkaline solution of Doripenem with aid of the appropriate acid. Surprisingly it has been found that the crystal shape and typical powder characteristics are influenced by the crystallization temperature.

At a temperature of about 20 °C a powder is obtained having a particle size distribution with $d_{0.1}$ of about 5 - 6 μm, $d_{0.5}$ of about 15 - 25 μm, respectively $d_{0.9}$ of about 40 - 60 μm, and a specific surface area of 1.7 - 3.0 $m^2/g$ suitable for powder filling without the need of additional aseptic handling like milling or sieving of the drug substance due to the presence of small particles and larger crystals. Avoiding any additional aseptic handling after crystallization is advantageously from sterile production point of view due to the limitation of non-sterile risks. However, if desired, a milling/sieving step may be included in the manufacturing. Doripenem Monohydrate with the disclosed particle size distribution shows advantageous filling properties as only a low tendency of dust formation is observed. Additionally an efficient production process by using 1-shot filling is possible.

Marketed Doripenem is milled. The process disclosed in EP 1886682 results in big crystals which have to be milled for an efficient powder filling step and a quick dissolution.

Surprisingly when performing the crystallization at a temperature of about 0 °C to 5 °C, very regular needles are formed with $d_{0.1}$ of about 7 μm, $d_{0.5}$ of about 24 μm respectively $d_{0.9}$ of about 82 μm and a specific surface area of 3.2 $m^2/g$ . Regular needles of Doripenem Monohydrate are advantageous as regular crystals are easily milled to the desired particle size distribution.

The aspect ratio (AR) of the needles obtainable at about 0 °C to 5 °C were determined by image analysis. The aspect ratio of a particle is the ratio of its longer dimension to its shorter dimension. The results of aspect ratio AR

calculated by the equitation a/b ( a = length, b = breath of the particles of the particles were determined to be typically as follows:

| a/b | % |
|---|---|
| 1-2 | 53.2 |
| 2-4 | 37.0 |
| 4-6 | 7.7 |
| 6-8 | 1.4 |

The aspect ratio was determined using a Zeiss Axioplan ( D-Oberkochen) equipped with an F-View II camera ( Olympus, Austria, A.-Vienna). The magnification was 200 and a phase contrast illumination was applied. The images (n= 100) are collected by an computer operated using software Cell F (Olympus Austria, A-Venna).

Samples were prepared by adding a weighted amount ( 30 ± 5 mg ) to a 500 mL glass vial. To this vial was added 2 mL suspending media (1 % SPAN 85). 60 seconds sonification was implemented to break agglomerates. A small volume was then collected via pipette from the suspension to a microscope slide prepared with one droplet Paraffinium liquidum. Both droplets are mixed well using a spatula.

The present invention is illustrated in the following examples, which should not be construed as limiting.

**Examples**

The X-ray powder diffraction patterns (XRPD) were collected on a Unisantis XMD 300 X-ray powder diffractometer with a position sensitive detector in parallel beam optics using the following acquisition conditions: tube anode: Cu , 40 kV, 0,8 mA, 3 - 43° theta/2theta; simultaneous detection of regions of 10° per step with detector resolution 1024, counting time 300 seconds per step. The samples were measured at room temperature in a standard sample holder on a rotating sample spinner. A typical precision of the 2-theta values is in the range of ± about 0.2° 2-theta. Thus a diffraction peak that appears at 5.0° 2-theta can appear between 4.8 and 5.2° 2-theta on most X-ray diffractometers under standard conditions.

The Infrared spectra (IR) were collected on a MKII Golden Gate™ Single Reflection Diamond ATR (attenuated total reflection) cell with a Bruker Tensor 27 FTIR spectrometer with 4 $cm^{-1}$ resolution at ambient conditions. To collect a spectrum a spatula tip of the sample was applied to the surface of the diamond in powder form. Then the sample was pressed onto the diamond with a sapphire anvil and the spectrum was recorded. A spectrum of the clean diamond was used as background spectrum. A typical precision of the wavenumber values is in the range of about ± 2 $cm^{-1}$. Thus, an infrared peak that appears at 1716 $cm^{-1}$ can appear between 1714 and 1718 $cm^{-1}$ on most infrared spectrometers under standard conditions.

Analytical method:

HPLC-Standard Method:

HPLC system : e.g. Agilent 1100
Eluent system : Ammoniumphosphate buffer / ACNL (pH 6.9)
Solvent : Deionized water
Column : XBridge C18, 2.5 μm, 4.6 x 50 mm
System : gradient
Flow rate : 1.5 mL/min
Oven temperature : 30 °C
Injection volume : 4 μL
Stop time : 25 min (Post-time 5 min)
Detection ; lambda = 210 nm
(Attenuation 2000 mAU for Agilent 1100 detectors)
Autosampler : 5 °C
Gradient:

| Min | 0 | 1.5 | 17 | 20 |
|---|---|---|---|---|
| %B | 0 | 3 | 26 | 100 |

Preparation of the solutions:

Reference stock solution (= reference solution for assay determination):

Dissolve 30 to 35 mg of the Doripenem Monohydrate working standard, weighed accurately to 0.01 mg, in solvent in a 10-mL volumetric flask. Fill to the mark with solvent and shake well.

Reference solution ( = 1 %; reference solution for determination of related substances):

Pipette 1.0 mL of the reference stock solution into a 100-mL volumetric flask, fill to the mark with solvent and shake well.

Test solution:

Dissolve 30 to 35 mg of the substance to be tested, weighed accurately to 0.01 mg, in solvent in a 10-mL volumetric flask. Fill to the mark with solvent and shake well.

Calculation:

Related Substance:

The content of the related substance ring-opened Doripenem (RO-DORI) was calculated in % by forming the product of measured peak area (mV·min) of RO-DORI in the chromatogram of the test solution, of the initial mass of the Doripenem working standard in the reference solution and the content of Doripenem in the working standard, and dividing it by the product of the initial mass of the test substance, of the peak area of the standard in the reference solution (= 1 %), and the dilution factor.

Weight-%:

$$\frac{m_R * A_T * C}{m_T * A_R * 100} = \% \text{ of related substance (RO-DORI)}$$

$m_T$ = initial mass of the substance to be tested in mg
$m_R$ = initial mass of the Doripenem working standard in the reference stock solution in mg
$A_T$ = peak area of related substance (RO-DORI) in the chromatogram of the test solution
$A_R$ = peak area of Doripenem in the chromatogram of the reference solution (= 1 %)
C = content of Doripenem in the working standard in % (95.7 % as is acc. to declaration)
100 = dilution factor from reference stock solution to reference solution

Example for calculation of RO-DORI in Example 7:

$$\frac{32.11\ mg * 0.053\ (mV * min) * 95.7\ \%}{33.76\ mg * 0.434\ (mV * min) * 100} = 0.111\ \%$$

Assay:

The content of Doripenem was calculated in % by forming the product of measured peak area (mV·min) of DORI in the chromatogram of the test solution, of the initial mass of the Doripenem working standard in the reference solution and the content of Doripenem in the working standard, and dividing it by the product of the initial mass of the

test substance and the peak area of the standard in the reference stock solution (= 100 %).

Weight-%:

$$\frac{m_R * A_T * C}{m_T * A_R} = \% \text{ of Doripenem}$$

$m_T$ = initial mass of the substance to be tested in mg
$m_R$ = initial mass of the Doripenem working standard in the reference stock solution in mg
$A_T$ = peak area of Doripenem in the chromatogram of the test solution
$A_R$ = peak area of Doripenem in the chromatogram of the reference stock solution (= 100 %)
C = content of Doripenem in the working standard in % (95.7 % as is acc. to declaration)

Example for calculation of DORI in Example 7:

$$\frac{32.11 \text{ mg} * 45.251 \text{ (mV * min)} * 95.7 \%}{33.76 \text{ mg} * 43.131 \text{ (mV * min)}} = 95.497 \%$$

Relative retention times:

| | Relative Retention Time |
|---|---|
| **RO-DORIPENEM** | 0.16 |
| Doripenem | **1.00** |

**Example 1:** Crystallization of Doripenem Monohydrate at 5 °C

3.514 g Doripenem Monohydrate were suspended in 63 mL $H_2O_{delon}$ at 5 °C. pH 10.0 ± 0.1 was adjusted by the addition of approximately 1.4 mL NaOH (20 %), whereas a clear solution was obtained. The solution was stirred at 5.0 ± 1.0 °C while approximately 2.8 mL HCl (10 %) were added to adjust pH 5.0 ± 0.1. Within about 5 minutes a white solid crystallized and the obtained suspension was stirred for 2 hours at 5.0 ± 1.0 °C. Then the suspension was cooled to 3 °C. After stirring the mixture at the same temperature for 2 hours 35 mL cold isopropanol were added over a period of about 0.5 hours. The slurry was further stirred for 17.5 hours at 3 °C before the solid was filtered off, washed with 7 mL isopropanol (80 %) and dried at 50 °C under vacuum for 24 hours (96.2 w% Doripenem, 0.1 w% RO-Doripenem). Typical water content (exp. KF titration) 4.0 - 4.5 %.

XRPD [2-theta angles]: 10.8 ± 0.2°, 12.9 ± 0.2°, 14.9 ± 0.2°, 15.8 ± 0.2°, 16.5 ± 0.2°, 19.5, ± 0.2°, 20.4 ± 0.2°, 20.9 ± 0.2°, 22.0 ± 0.2°, 23.8 ± 0.2°, 26.0 ± 0.2°, 28.1 ± 0.2°. 29.0 ± 0.2°, 31.6 ± 0.2°; FTIR: 3526 ± 2 $cm^{-1}$, 3224 ± 2 $cm^{-1}$, 1710 ± 2 $cm^{-1}$, 1631 ± 2 $cm^{-1}$, 1565 ± 2 $cm^{-1}$, 1455 ± 2 $cm^{-1}$, 1385 ± 2 $cm^{-1}$, 1349 ± 2 $cm^{-1}$, 1320 ± 2 $cm^{-1}$, 1263 ± 2 $cm^{-1}$, 1161 ± 2 $cm^{-1}$, 1088 ± 2 $cm^{-1}$, 929 ± 2 $cm^{-1}$, 763 ± 2 $cm^{-1}$;

**Example 2:** Crystallization of Doripenem Monohydrate at 10 °C

3.508 g Doripenem Monohydrate were suspended in 63 mL $H_2O_{delon}$ at 10 °C, pH 10.0 ± 0.1 was adjusted by the addition of approximately 1.4 mL NaOH (20 %), whereas a clear solution was obtained. The solution was stirred at 10.0 ± 1.0 °C while approximately 2.8 mL HCl (10 %) were added to adjust pH 5.0 ± 0.1. Within about 5 minutes a white solid crystallized and the obtained suspension was stirred for 2 hours at 10.0 ± 1.0 °C. Then the suspension was cooled to 3 °C. After stirring the mixture at the same temperature for 2 hours 35 mL cold isopropanol were added over a period of about 1 hour. The slurry was further stirred for 17 hours at 3 °C before the solid was filtered off, washed with 7 mL isopropanol (80 %) and dried at 50 °C under vacuum for 5.5 hours (96.1 w% Doripenem, 0.1 w% RO-Doripenem).

**Example 3:** Crystallization of Doripenem Monohydrate at 20 °C

4.001 g Doripenem Monohydrate were suspended in 72 mL $H_2O_{delon}$ at 20 °C. pH 10.0 ± 0.1 was adjusted by the addition of approximately 1.6 mL NaOH (20 %), whereas a clear solution was obtained. The solution was stirred at 20.0 ± 1.0 °C while approximately 3.2 mL HCl (10 %) were added to adjust pH 5.0 ± 0.1. Within about 5 minutes a white solid crystallized and the obtained suspension was stirred for 2 hours at 20.0 ± 1.0 °C. Then the suspension was cooled to 3 °C. After stirring the mixture at the same temperature for 2 hours 40 mL cold isopropanol were added over a period of about 1 hour. The slurry was further stirred for 16.5 hours at 3 °C before the solid was filtered off, washed with 8 mL isopropanol (80 %) and dried at 50 °C under vacuum for 23.5 hours (95.8 w% Doripenem, 0.1 % RO-Doripenem).

**Example 4:** Crystallization of Doripenem Monohydrate at 25 °C

4.012 g Doripenem Monohydrate were suspended in 72 mL $H_2O_{delon}$ at 25 °C. pH 10.0 ± 0.1 was adjusted by the addition of approximately 1.6 mL NaOH (20 %), whereas a clear solution was obtained. The solution was stirred at 25.0 ± 1.0 °C while approximately 3.2 mL HCl (10 %) were added to adjust pH 5.0 ± 0.1. Within about 5 minutes a white solid crystallized and the obtained suspension was stirred for 3 hours at 25.0 ± 1.0 °C. Then the suspension was cooled to 3 °C. After stirring the mixture at the same temperature for 2 hours 40 mL cold isopropanol were added over a period of about 1 hour. The slurry was further stirred for 16 hours at 3 °C before the solid was filtered off, washed with 8 mL isopropanol (80 %) and dried at 50 °C under vacuum for 7 hours (95.6 w% Doripenem, 0,1 w% RO-Doripenem).

**Example 5:** Crystallization of Doripenem Monohydrate according to the present invention

9.80 g Doripenem Monohydrate were suspended in 180 mL $H_2O_{delon}$. (pH 5.01, T = 20.1 °C). To the suspension about 4 mL NaOH (20 %) were added, whereas a clear solution was obtained (pH 10.0 T = 20.0 °C). Thereafter about 8 ml HCl (10 %) were added to the solution (pH 5.1, T = 20.0 °C). Within about 10 minutes a white solid crystallized and the suspension was further stirred at 20 °C for 2 hours. Then the suspension was cooled to 3 °C within about one hour and further stirred at this temperature for 2 hours. Afterwards 100 mL isopropanol were slowly added to the suspension over a time period of about 2 hours. After the antisolvent addition the suspension was further stirred at 3 °C for 15.5 hours. Finally the solid was filtered off, washed with isopropanol (80 % by volume) and dried at 50 °C under vacuum for about 8 hours to obtain 8.49 g (87 % yield) of crystalline Doripenem Monohydrate.

**Example 6:** Decomposition of Doripenem in aqueous solution at pH 10

The decomposition of Doripenem in aqueous solution at pH 10 was examined by dissolving 47.71 mg of Doripenem Monohydrate in 10 mL of ammonium phosphate buffer at pH 10 (prepared by dissolving 2.0 mL of 85 % phosphoric acid in 800 mL of water, adjusting the pH with 25 % aqueous ammonia to pH 10, and adding up to a total volume of 1000 mL with water), and repeated injection of 4.0 µL of this test solution into the H PLC system over a period of 9 hours. Analysis of data was performed according to the method described.
Doripenem was stable over a period of 9 hours. Figure 1 shows the Doripenem decomposition curve at pH 10.

**Example 7:** Crystallization process of Doripenem Monohydrate according to the present invention

1.992 g Doripenem Monohydrate were suspended in 36 mL $H_2O_{delon}$ at 20 °C. pH 10.0 ± 0.1 was adjusted by the addition of approximately 0.8 mL NaOH (20 %), whereas a clear solution was obtained. The solution was stirred at 20.0 ± 1.0 °C while approximately 1.6 mL HCl (10 %) were added to adjust pH 5.0 ± 0.1. Within about 5 minutes a white solid crystallized and the obtained suspension was stirred for 2 hours at 20.0 ± 1.0 °C. Then the suspension was cooled to 3 °C. After stirring the mixture at the same temperature for 2 hours 20 mL cold isopropanol were added over a period of about 0.5 hours. The slurry was further stirred for 18 hours at 3 °C before the solid was filtered off, washed with 4 mL isopropanol (80 %) and dried at 50 °C under vacuum for 7.5 hours (95.5 w% Doripenem, 0.1 w% RO-Doripenem).

**Example 8:**

2.951 g of Doripenem Monohydrate were suspended in 54 ml $H_2O_{delon}$ and 30 ml of isopropanol at 20°C. A pH of 10 ± 0.1 was adjusted by the addition of approximately 1,2 ml of 20% NaOH whereas a clear solution was obtained. The pH was adjusted to pH 5.0 ± 0.1 by addition of 2.4 ml of 10% HCl. Within about 5 minutes a white solid crystallized. The suspension was allowed to stir at 20° C for 3 hours and the suspension was then cooled to 3°C ± 1°C within one hour and stirred for approximately 19 hours at this temperature.
The crystals were then isolated by filtration and washed with 6 mL isopropanol (80%).

The crystals were then dried under vacuum at 50°C for 8 hours.

Yield: 2,45 g of Doripenem Monohydrate (0.1 w% RO-Doripenem).

**Reference Example 1:** Crystallization process of Doripenem Monohydrate according to Example 1 of EP 1270575

2.005 g Doripenem (Doribax®lot0045) were suspended in 36 ml $H_2O_{delon}$ at 20 °C. The suspension was dissolved at 55 °C. Afterwards the solution was cooled down to 20.0 ± 1.0 °C, whereas at 30 °C a white solid started to crystallize. The solution was stirred at 20.0 ± 1.0 °C for 2 hours. Then the suspension was cooled to 3 °C. After stirring the mixture at the same temperature for 2 hours 20 ml cold isopropanol were added over a period of about 1 hour. The slurry was further stirred for 15.5 hours at 3 °C before the solid was filtered off, washed with 4 ml isopropanol (80 %) and dried at 50 °C under vacuum for 15.5 hours (94.3 w% Doripenem, 0.20 w% RO-Doripenem).

## Claims

1. Doripenem Monohydrate with a content of not more than 0.1 % (by weight) of ring-opened Doripenem (RO-Doripenem) of formula

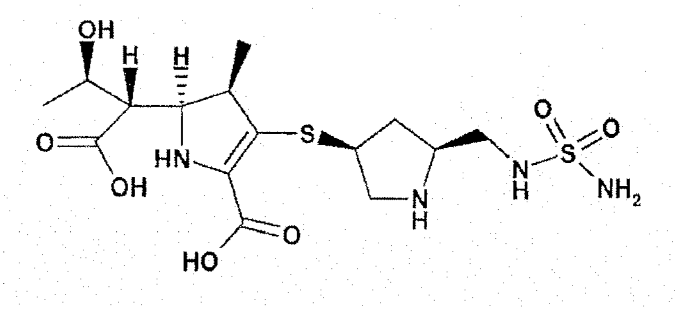

determined by HPLC at 210 nm against a solution of reference standard of Doripenem representing a content of 1 % by weight.

2. A crystallization process for Doripenem Monohydrate comprising the steps of

(a) suspending Doripenem in water,
(b) dissolving Doripenem at pH of 8-12 by the addition of a base,
(c) optionally filtering the solution through a micron filter,
(d) crystallizing Doripenem at pH 3 - 6 by the addition of an acid,
(e) optionally adding an organic solvent and
(f) isolating and drying the obtained crystals.

3. A crystallization process for Doripenem Monohydrate according to claim 2 comprising the steps of

(a) suspending Doripenem in water,
(b) dissolving Doripenem at pH 10 by the addition of a base,
(c) optionally filtering the solution through a micron filter,
(d) crystallizing Doripenem at pH 5 by the addition of an acid
(e) optionally adding an antisolvent and
(f) isolating and drying the obtained crystals.

4. Doripenem Monohydrate with a content of not more than 0.1 % of RO-Doripenem (by weight-%) determined by HPLC at 210 nm against a solution of reference standard of Doripenem representing a content of 1 % by weight according to claim 2 or 3.

5. A crystallization process for Doripenem Monohydrate according to claim 2 and claim 3, wherein the organic solvent is selected from isopropanol or a $C_1$-$C_4$ alcohol.

6. A process according to claim 5 wherein the organic solvent is isopropanol.

**7.** A crystallization process for Doripenem Monohydrate according to claim 2 or 3 wherein the base is selected from an ammonium source, an organic amine, an alkali or earth alkali hydroxide, alkali or earth alkali carbonate or bicarbonate.

**8.** A crystallization process for Doripenem Monohydrate according to claim 7, wherein the base is sodium hydroxide.

**9.** A crystallization process for Doripenem Monohydrate according to claim 2 or 3 wherein the acid is selected from an organic acid or a mineral acid.

**10.** A crystallization process for Doripenem Monohydrate according to claim 9, wherein the acid is hydrochloric acid.

Figure1

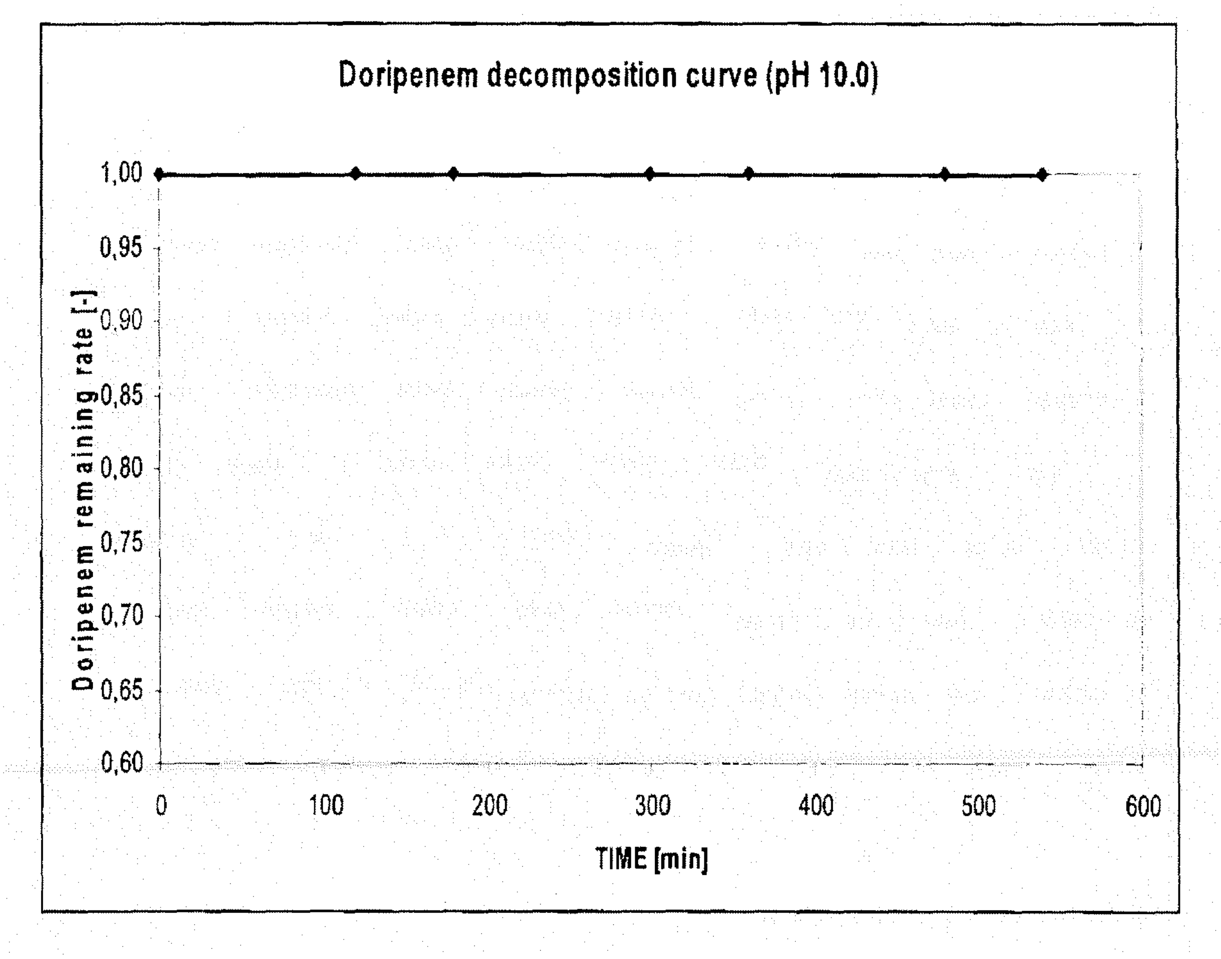
Doripenem decomposition curve (pH 10.0)
1,00
0,95
0,90
0,85
0,80
0,75
0,70
0,65
0,60
Doripenem remaining rate [-]
0
100
200
300
400
500
600
TIME [min]

Europäisches Patentamt
European Patent Office
Office européen des brevets

# EUROPEAN SEARCH REPORT

Application Number
EP 10 16 9837

**DOCUMENTS CONSIDERED TO BE RELEVANT**

| Category | Citation of document with indication, where appropriate, of relevant passages | Relevant to claim | CLASSIFICATION OF THE APPLICATION (IPC) |
|---|---|---|---|
| X | EMEA: "CHMP ASSESSMENT REPORT" 20080101, no. EMEA/309032/2008, 1 January 2008 (2008-01-01), pages 1-60, XP007914813 * page 6 - page 7 * ----- | 1,4 | INV. C07D477/20 A61K31/407 A61P31/04 |
| X | PSATHAS PETROS A ET AL: "Stability of doripenem in vitro in representative infusion solutions and infusion bags." CLINICAL THERAPEUTICS NOV 2008 LNKD- PUBMED:19108795, vol. 30, no. 11, November 2008 (2008-11), pages 2075-2087, XP002600044 ISSN: 0149-2918 * page 2078 - page 2079 * ----- | 1,4 | |
| A | D. BLACK: "the d-zone" DRUG THERAPY TODAY, vol. 36, no. sup 3, 31 August 2007 (2007-08-31), XP007914837 * page S2 * ----- | 1 | TECHNICAL FIELDS SEARCHED (IPC) |
| Y | NISHINO Y ET AL: "Practical Large-scale Synthesis of Doripenem: A Novel 1beta-Methylcrabapenem Antibiotic" ORGANIC PROCESS RESEARCH AND DEVELOPMENT, CAMBRIDGE, GB, vol. 7, 24 September 2003 (2003-09-24), pages 846-850, XP002396087 * page 850 * ----- | 2-10 | C07D A61K A61P |
| Y | WO 2007/031858 A2 (ORCHID CHEMICALS & PHARM LTD [IN]; SURULICHAMY SENTHILKUMAR [IN]; SEKA) 22 March 2007 (2007-03-22) * claim 1 * ----- | 2-10 | |
| | -/-- | | |

The present search report has been drawn up for all claims

| Place of search | Date of completion of the search | Examiner |
|---|---|---|
| The Hague | 10 September 2010 | Skulj, Primoz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 10 16 9837

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 2009/118680 A1 (RANBAXY LAB LTD [IN]; GEORGE VINOD [IN]; MEERAN HASHIM NIZAR POOVANATH) 1 October 2009 (2009-10-01)<br>* the whole document *<br>----- | 2,3,5-7, 9,10 | |
| | | | TECHNICAL FIELDS SEARCHED (IPC) |

The present search report has been drawn up for all claims

| Place of search | Date of completion of the search | Examiner |
|---|---|---|
| The Hague | 10 September 2010 | Skulj, Primoz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 10 16 9837

This annex lists the patent family members relating to the patent documents cited in the above-mentioned European search report.
The members are as contained in the European Patent Office EDP file on
The European Patent Office is in no way liable for these particulars which are merely given for the purpose of information.

10-09-2010

| Patent document cited in search report | Publication date | Patent family member(s) | Publication date |
|---|---|---|---|
| WO 2007031858 A2 | 22-03-2007 | AU 2006290416 A1 | 22-03-2007 |
| | | EP 1934221 A2 | 25-06-2008 |
| | | JP 2009508840 T | 05-03-2009 |
| | | NZ 566460 A | 30-04-2010 |
| | | US 2009264643 A1 | 22-10-2009 |
| WO 2009118680 A1 | 01-10-2009 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 557122 A
- EP 758651 A
- EP 1270575 A
- EP 1886682 A
- WO 2006117763 A

### Non-patent literature cited in the description


- **Zhanel GG et al.** Comparative review of the carbapenems. *Drugs,* 2007, vol. 67 (7), 1027-1052
- **Brown SD ; Traczewski MM.** Comparative in vitro antimicrobial activity of a new carbapenem, Doripenem: tentative disc diffusion criteria and quality control. *J. Antimicrob. Chemother,* June 2005, vol. 55 (6), 944-949
- **Pillar CM et al.** In vitro activity of Doripenem, a carbapenem for the treatment of challenging infections caused by gram-negative bacteria, against recent clinical isolates from the United States. *Antimicrob. Agents Chemother,* December 2008, vol. 52 (12), 4388-4399
- **Jones RN et al.** Doripenem (S-4661), a novel carbapenem: comparative activity against contemporary pathogens including bactericidal action and preliminary in vitro methods evaluations. *J. Antimicrob. Chemother,* July 2004, vol. 54 (1), 144-154
- **Cirillo I et al.** Disposition, metabolism, and excretion of [14C]Doripenem after a single 500-milligramm intravenous infusion in healthy men. *Antimicrob. Agents Chemother,* 05 October 2008, vol. 2 (10), 3478-3483